# EUROPEAN PATENT SPECIFICATION

(11) **EP 2 520 315 B1**
(45) Date of publication and mention of the grant of the patent: **30.09.2020**
(21) Application number: 10840300.7
(22) Date of filing: 17.12.2010
(51) Int. Cl.: A61L 9/22, B03C 3/40, B03C 3/38, B03C 3/08, A61L 9/20, B03C 3/41, B03C 3/47, B03C 3/60, H05H 1/24, B01D 53/32

(54) **METAL BELT-PLATE STRUCTURE REACTOR**
REAKTOR MIT METALLRIEMEN- UND PLATTENSTRUKTUR
RÉACTEUR À STRUCTURE DE COURROIES-PLAQUES MÉTALLIQUES

(30) Priority: 31.12.2009 CN 200910263798
(43) Date of publication of application: 07.11.2012
(60) Divisional application: 20152500.3
(73) Proprietor: Zhejiang Tianqing Environmental Protection Technology Co., Ltd., Zhejiang Province (CN)
(72) Inventor: ZHOU, Yunzheng, Shanghai 200434 (CN); FU, Jianwei, Shanghai 200434 (CN); ZHOU, Jin, Shanghai 200434 (CN)
(74) Representative: Laufhütte, Dieter
(86) International application number: PCT/CN2010/002083
(87) International publication number: WO 2011/079510

(56) References cited:
- EP-A1- 1 829 615
- CN-A- 101 920 034
- CN-A- 101 922 750
- CN-U- 201 644 086
- CN-Y- 2 424 827
- JP-A- S 527 075
- JP-A- 2004 267 959
- JP-Y1- S4 011 117
- US-A- 4 074 983
- US-A- 4 381 927
- US-A- 4 472 174

## Description

### BACKGROUND OF THE INVENTION

### 1. Technical Field

The present invention belongs to the technical field of air sterilization and purification and in particular relates to a metal belt-plate structured reactor.

### 2. Description of Related Art

The non-thermal plasma reactor of the existing air sterilizing and purifying device consists of positive electrodes, negative electrodes, insulation supports and a housing case. The positive electrode has several major structures, such as the metal wire, saw tooth and needle tip.

At normal temperature and normal pressure, the discharge positive electrode with an oxidization-resistant thin metal wire, saw-tooth or needle tip-structured discharge positive electrode and a negative electrode made from an anti-oxidation metal plate are used for generating non-thermal plasma by means of corona discharge to sterilize and purify the air, and the thin metal wire performs uniform corona discharge, generates high-concentration non-thermal plasma, and is therefore a preferable option.

The plasma reactors with the thin metal wire-structured discharge positive electrode for air sterilization and purification were available in the market, but disappeared in recent three years to be replaced by reactors with saw-tooth or needle tip-structured discharge positive electrodes. The existing filtering absorption, high-voltage static, ozone, ultraviolet and TiO₂ photo-catalysis type air sterilizing and purifying devices have advantages and disadvantages in the three aspects of sterilization and purification, toxic organic compound decomposition and respirable particle filtration. For example, the filtering absorption and high-voltage static type air sterilizing and purifying devices are not good at air sterilization and purification and toxic organic compound decomposition, while the ozone, ultraviolet and TiO₂ photo catalysis-type air sterilizing and purifying devices are unable to remove respirable particles. The above products are widely adopted by hospitals, office buildings, shopping malls and public places of entertainment, even if their air sterilizing and purifying functions are incomplete. The majority of the food factories, pharmaceutical plants and semiconductor IC manufacturers adopt the FFU air filter unit. The FFU air filter unit has a good air sterilizing and purifying effect at the beginning and occupies a large market share, even through it has the defects of large energy consumption, high noise and high maintenance costs and may cause secondary pollution.

The fundamental reason of this phenomenon is the unreasonable design of the non-thermal plasma reactor: although the thin metal wire forming the discharge positive electrode of the non-thermal plasma reactor generates plasma of high concentration, it is susceptible to burning. Therefore, the discharge positive electrode is usually made of stainless steel and structured as a saw-tooth or needle tip. The saw-tooth or needle tip-shaped structure is insusceptible to burning, but discharge occurs at the tips to form a discharge column, and a violet and blue light line with a diameter of about 0.22mm is visible between the positive electrode and the negative electrode in a dark room, which is the phenomenon of non-uniform discharge in the air. The plasma near the violet and blue light line is dense, so the oxygen and nitrogen in the air are susceptible to activation to generate ozone, nitric oxides, etc., while the plasma away from the violet and blue light line is thin, so the air sterilizing and purifying effect is poor. The sterilizing effect of this kind of reactor is restrained by the concentrations of ozone and nitric oxides, which is the biggest problem for the technicians in this field. Furthermore, there is another major defect: after working for several months, the saw-tooth or needle tip-shaped discharge point becomes blunt because of the sputtering effect. Along with increasing the curvature radius of the positive electrode and raising the discharge inception voltage, the discharge current is reduced, and then the air sterilizing and purifying efficiency is certainly lowered. This decline phenomenon is invisible, it is difficult to find the reduction of the plasma concentration, and although the reactor surface still works, it is almost ineffective. If this kind of plasma reactor is used in the operating room and the intensive care unit of hospitals, unqualified sterilization may generate bacteria and viruses and cause infection and operation failure.

For example, the Chinese patent -Building Block-type Narrow-spacing Electrostatic Field Device, application No. 200710038821.4, indicates that "the defect that the thin wire is easy to break greatly lowers the reliability of the device" in the first page of its description. The technical scheme of this invention provides a building block-type narrow-spacing electrostatic field device, comprising a discharge electrode (positive electrode) and a dust collector (negative electrode), wherein the discharge electrode and the dust collector are arrayed in parallel at an interval, the two ends of the discharge electrode are connected with discharge electrode connections, the lower part of the discharge electrode is saw tooth-like, the upper part of the discharge electrode is tubular, the saw tooth-like discharge electrode end and the dust collector form a dust collection region; the two ends of the dust collector are connected with dust collection connections; and the discharge electrode connections and the dust collection connections are connected with insulators, respectively.

In this technical scheme, although the electrostatic field device has a long service life, ozone is inevitably generated when the oxygen in the air near the discharge point is activated because of the extremely non-uniform discharge of the saw tooth-like discharge electrode; furthermore, the "decline phenomenon" of the point discharge always exists.

The Chinese patent-Ionization-type Air Purifier, application No. 200610024299.X has the following statement in the claims: An ionization-type air purifier consists of several positive electrodes and negative electrodes which are identical in length and respectively fixed on insulation boards to form a rectangular electric field, the positive electrodes are arrayed in rows, and the two ends thereof are vertically fixed on the insulation boards; anticorrosive and very tough metal wires with diameters of 0.05-0.2mm are used as the positive electrodes, and copper or stainless steel tubes with a diameter of 8-30cm are used as the positive electrodes.

The positive electrodes of the ionization-type air purifier are made of anticorrosive and very tough metal wires and therefore generate dense plasma, but the two ends of the positive electrode are respectively fixed on two insulation boards vertically; due to the micro-discharge effect generated between the positive electrodes and the insulation boards, the metal wires of the positive electrodes are burned out for just a few months. Actually, the scheme has no practical significance, and this is why this ionization-type air purifier has disappeared over the past three years.

In the prior art, the root cause for the thin metal wire forming the positive electrode of the plasma reactor to easily burn out is the lack of a micro-discharge effect; the physical factors are also unclear, and thus the technical scheme preventing the micro-discharge effect cannot be figured out. The majority of the manufacturers which produce air sterilizing and purifying devices having the plasma reactors with the saw-tooth or needle tip-structured positive electrodes at present adopt metal wires as the positive electrodes of the plasma reactors and now face the problem that ozone and nitric oxide concentrations exceed the standard when the air sterilizing and purifying effect is achieved. That is to say, when the air sterilization test is not qualified, and ozone smell is terrible, and it is difficult to meet the provisions on ozone amounts ≤0.16mg/m³ in the Indoor Air Quality Standard GB/T18883-2002.

Even worse, the plasma reactor is also removed, and the needle tip or saw tooth-structured electrode is used as the electronic static absorption purifier which only absorbs the dust and is inferior to the plasma reactor in the capability of decomposing the toxic organics. The scheme of replacing the positive electrode made of thin metal wires by the saw-tooth or needle tip-structured electrode to exchange the sterilizing and purifying effect for the reliability and service life of the sterilizing and purifying device is a kind of technical bias.

US 4 318 927 A relates to an improved electrode arrangement for use in an electrostatic precipitator that aids the efficient collection and elimination of dusts and mists from industrial effluent gases before they are released to the atmosphere.

JP S52 7075 A provides an electric dust arrester, whose dust arresting efficiency has been highly improved.

JP S40 11117 Y1 exhibits a similar device.

US 4074 983 A relates to electrostatic precipitators and, more particularly, to a new, improved and more efficient wet electrostatic precipitator.

EP 1 829 615 A1 relates to an electric dust-collecting unit placed in a space of air stream of such a product as an air conditioner for removing dust and the like contained in the air being conditioned.

### BRIEF SUMMARY OF THE INVENTION

The object of the present invention is to overcome the mentioned defects and bias of the prior art and provide a metal belt-plate structured reactor which performs uniform corona discharge, generates high-concentration plasma, has high reliability and a long service life and can prevent the phenomenon of micro-discharge.

Experiments show that a reactor with a saw-tooth or needle tip-structured positive electrode performs corona discharge at the point, while a reactor with a positive electrode made of metal wires or metal belts performs corona discharge around the wires or belts, and uniformity and condensation the plasma generated under the same conditions are obviously better than that generated by the saw-tooth or needle tip-structured electrode. When corona discharge is performed at room temperature and normal pressure, the positive electrode is finally exhausted because of sputtering even if the micro-discharge phenomenon is voided. The experiments prove that a positive electrode made of metal belts and a positive electrode made of metal wires have the same corona discharge effects; however, the service life of the metal belts is several times that of the metal wires, so the metal belt positive electrode-negative electrode structured reactor is preferable in the present invention.

To fulfill the mentioned aim, the root cause causing the "thin metal wire positive electrode of the plasma reactor to easily burn out" must be found first. Research and analysis found that, to solve the problem that the high voltage between the positive and negative electrodes reaches tens of thousands of volts, the majority of the technicians adopt supports made of insulation materials such as plastic, polymethyl methacrylate and epoxy resin to fix the positive and negative electrodes. A few people add a thin spring made of the metal wires between the insulation support and the electrode. Studies and experiments show that insulation materials with a high dielectric constant can isolate the high-potential positive and negative electrodes well, but have a fatal defect that a micro-discharge phenomenon appears. The strong electric field is formed around the positive electrode, and by the catalytic action of the plasma, the micro-discharge appears locally in the area where the positive electrode made of a metal wire contacts the insulation materials. The higher the dielectric constant is, the more severe the surface micro-discharge phenomenon is. To improve the sterilizing and purifying effect, raising the voltage of the external power supplies at the two electrodes of the plasma is a common method, which also correspondingly strengthens the polarity effect. The high-energy electrons generated by the micro-discharge phenomenon directly ionize and decompose the molecules of the insulation materials and the metal conductive materials to generate oxides and water. This is the root cause for the thin metal wire-structured discharge positive electrode of the plasma reactor easily burn out. Therefore, it is rational that plasma reactors with thin metal wire-structured discharge positive electrodes have disappeared over the past three years.

The inventor used the high-voltage pulse power supplies with specifications to make contrast tests on the non-thermal plasma reactors with wire, saw tooth or needle tip-structured positive electrodes by continuously electrifying them day and night in a closed space. The working currents of the reactors with the saw-tooth and needle tip-structured positive electrodes declined obviously only after the reactors worked for two weeks, the reactor with the wire-structured positive electrode had wire broken after 6 weeks, and the reactor with the belt-structured positive electrode was relatively stable. The bottoms of all the reactors are covered with a dark liquid; the surfaces of the insulators between the positive and negative electrodes had traces of yellow, dark creepage, and the surface of the stainless steel positive electrode in this area also was also corroded. The reactor with a thin spring between the insulation support and the metal wire had a similar problem, and the wire breakdown was just delayed by about two or three months. This is because the spring is not allowed to be too thick; otherwise, the metal wire is easily broken down by pulling or the spring will not work. Even springs made of stainless steel are inevitably corroded by the micro-discharge.

In the aging experiments, influenced by the micro-discharge which is generated by means of the interaction of the metal and the insulation material surface, a small part of the plasma is reduced because of the pollution caused by the micro-discharge, which reduces the working efficiency of the reactor, damages the structure of the reactor and shortens the service life. Studying the effect of the micro-discharge mechanism in the plasma reactor and providing a practical and feasible technical improvement scheme is the major objective of the present invention.

To fulfill the mentioned objective, the present invention provides a metal belt-plate structured reactor according to claim 1.

Preferably, the micro-discharge preventive conductor rails are provided with convex portions arrayed at an equal interval, and the two ends of the positive electrode are respectively fixed on the tops of the convex portions of the corresponding conduct rails; the vertically symmetrical convex portions of the micro-discharge preventive conductor rails are incorporated into one group, each micro-discharge preventive conductor rail has n groups of convex portions, and the top of each convex portion is provided with an outward elbow. The two ends of the positive electrodes are provided with stainless steel connecting frames, of which the centers are punched into square holes, and the elbows at the tops of the protruded portions are sleeved into the square holes of the stainless steel connecting frames. The upper and lower ends, close to the edge of the reactor housing, of the negative electrodes are respectively provided with a projected negative electrode fixing pin which is jointed and fastened with the groove at the corresponding position of the reactor housing; the two ends of each micro-discharge preventive conductor rail is provided with an insulation connection fixed in the corresponding mounting hole of the reactor housing. Airflow channels are formed on the upper and lower ends of the housing along the airflow direction.

Preferably, the micro-discharge preventive conductor rail has n groups of recessed portions arrayed at an equal interval, and the two ends of the positive electrode made of the metal belt are respectively fixed in the recessed portions of the corresponding micro-discharge conductor rail. The two ends of the micro-discharge preventive conductor rail are orthogonally fixed to and electrically connected with the four positive electrode metal supports on the four sides of the reactor. The edge of the positive electrode metal support is provided with several cylindrical fixing rings for micro-discharge preventive conductor rails, and the cylindrical fixing rings separate the micro-discharge preventive conductor rails at an equal interval. The upper and lower ends of each positive electrode metal support are respectively provided with an insulation connection fixed in the corresponding mounting hole on the reactor housing, and the insulation connections are fastened on the reactor housing by insulation connection fixing bolts. The upper and lower sides, close to the reactor housing, of the negative electrode are jointed and fastened with the reactor housing; and the airflow channels are formed on the front and rear ends of the housing along the airflow direction.

Preferably, the surface of the negative electrode is made from oxidized aluminum plate, and the two sides of the negative electrode are coated with nano-scaled TiO₂.

Preferably, the distance between the elbows on two vertically symmetrical projected portions in each group is designed according to the unipolar shielding effect, and then the distance between two metal belts of the adjacent positive electrodes ranges from 16-26 mm.

Preferably, the distance between the two adjacent micro discharge preventive conductor rails with grooves is designed according to the unipolar shielding effect, and then the distance between the metal belts of two adjacent positive electrodes ranges from 16-26 mm.

Preferably, the discharge distance between the positive and negative electrodes is designed to be 8-18mm.

According to the invention the positive electrode is made of high-resistance electrothermal nickel-chromium alloy belt or high-resistance electrothermal iron-chromium-aluminum alloy belt which is 1-2mm wide and 0.05-0.2mm thick.

Compared with the prior art, the present invention has the following advantages:
The positive electrode in the present invention is made of oxidized metal belts which are arrayed in parallel at an equal interval on the same plane, the negative electrode is made of aluminum or stainless steel plates, several micro-discharge preventive conductor rails made of the aluminum bars or stainless steel bars shall be approved according to the power requirements of the reactor, and the two ends of the positive electrode are respectively fixed on two corresponding micro-discharge preventive conductor rails. The two ends of the positive electrode are isolated by the micro-discharge preventive conductor rail, away from the fixing insulation connections and therefore, in comparison with the prior art, the micro-discharge effect is prevented. Each nickel-chromium alloy belt performs corona discharge stably in the strong direct current dielectric field to generate the high-concentration plasma. The defect that "the thin wire is easy to break" is overcome, and the technical bias of "scarifying the sterilizing and purifying effect to obtain the reliability and service life of the sterilizing and purifying device by replacing the positive electrode made of the thin metal wire by the saw-tooth or needle tip-structured discharge positive electrode" is corrected. Furthermore, the lead of the negative electrode of the external direct current power supply is connected with the ground wire of the metal housing of the plasma reactor, so the safety is guaranteed, and the electromagnetic shielding effect is better and meets the requirements for electromagnetic comparability.

The two sides of the micro-discharge preventive conductor rails and negative electrodes are precisely fixed on the housing, which ensures that the positive electrode is positioned at the middle of the two adjacent negative electrodes during integrated installation. In such a way, the discharge distance between the positive electrode and the negative electrode is always kept constant-this is a key to the reactor quality; the positive electrodes formed by several metal belts, the micro-discharge preventive conductor rails, the negative electrodes, and the reactor housing are connected integrally, and the whole structure is solid and has a good insulation property and a simple installation process.

The present invention has an additional benefit: when the anti-oxidation metal belt performs corona discharge in the reactor to cause sputtering which damages the electrode, the damaged part of the metal belt moves further away from the negative electrode, and then the discharge current is reduced automatically; on the contrary, the discharge current is increased automatically. Thus, the damaged part of the positive electrode is self-adjusted during work to prolong the service life, and an unexpected good effect is achieved.

The present invention provides a metal belt-plate structured reactor which performs uniform corona discharge, generates high-concentration plasma, has high reliability, a long service life, solid whole structure, a good insulation property and a simple installation process and can prevent the micro-discharge phenomenon. Once the mentioned objective is fulfilled, the defects and bias in the prior art are overcome.

The metal belt-plate structured reactor is the core part of the plasma air sterilizing and purifying device and externally connected with a high-voltage pulse power supply, the positive electrode of the output end of the pulse power supply is electrically connected with the positive electrode of the plasma reactor, the negative electrode of the output end of the pulse power supply is electrically connected with the negative electrode (housing) of the plasma reactor, and the plasma reactor and the fan are positioned between the air inlet and air outlet of the air sterilizing and purifying device; then, the indoor air circulating for 8-10 cycles in the plasma reactor can be sterilized and purified. Test reports indicate that the reactor applied to the plasma air sterilizing and purifying device has a good broad-spectrum sterilization effect on the following bacteria and viruses: staphylococcus aureus, colibacillus, bacillus subtilis, Candida albicans, mould, mycoplasma, hepatitis B and flu. Meanwhile, it also has the functions of absorbing respirable particles, removing particular smell and degrading formaldehyde, smog and organic waste gases such as TVOC. According to the statements of the reports, when the reactor works for 30 min in a room with an area of 100m³, the rate of killing the staphylococcus albus and staphylococcus aureus is 99.9%, the total colony number is less than or equal to 200cfu/m3, the particular smell removal rate is greater than or equal to 96%, the quantity of the suspended particles is less than or equal to 350/L (Φ≥0.5µm), the formaldehyde removal rate reaches 98%, and the amount of ozone residing in the air is less than or equal to 0.02mg/m³. The energy conservation is conspicuous: the power consumed by the reactor to meet the type-II hospital environmental sterilization standards when working in a 100m³ room is 7-8 W, while the ultraviolet or ozone-type air purifiers at least consume 160 W of power to achieve the same effect.

Therefore, for those skilled in this field, the present invention can achieve good unexpected technical effects in comparison with the prior art.

### BRIEF DESCRIPTION OF THE SEVERAL VIEWS OF THE DRAWINGS

Figure 1 is a three-dimensional view with a local sectional side of one embodiment of the present invention;
Figure 2 is a structural view of an amplified micro-discharge preventive conductor rail in figure 1 of the present invention;
Figure 3 is a schematic view of the corona discharge of the present invention;
Figure 4 is a three-dimensional view of another embodiment of the present invention.

### Descriptions of the signs of major parts in the attached drawings:

- 1-: plasma reactor
- 2-: direct current high-voltage power supply
- 5-: inflow air
- 6-: outflow air
- 101-: positive electrode
- 102-: negative electrode
- 103-: micro-discharge preventive conductor rail
- 104-: positive electrode metal support
- 105-: conductor rail insulation support
- 106-: fixing bolt
- 107-: fixing ring
- 108-: housing
- 109-: projected portion
- 110-: stainless steel connecting frame
- 111-: negative electrode fixing pin

### DETAILED DESCRIPTION OF THE INVENTION

### Embodiment 1:

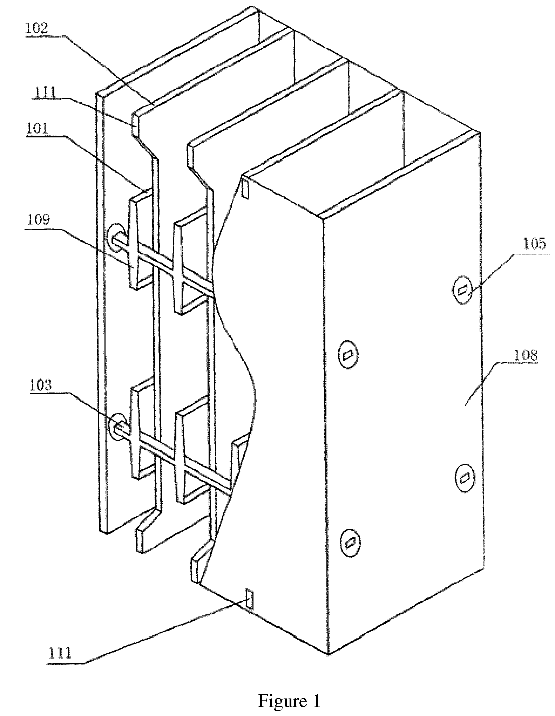

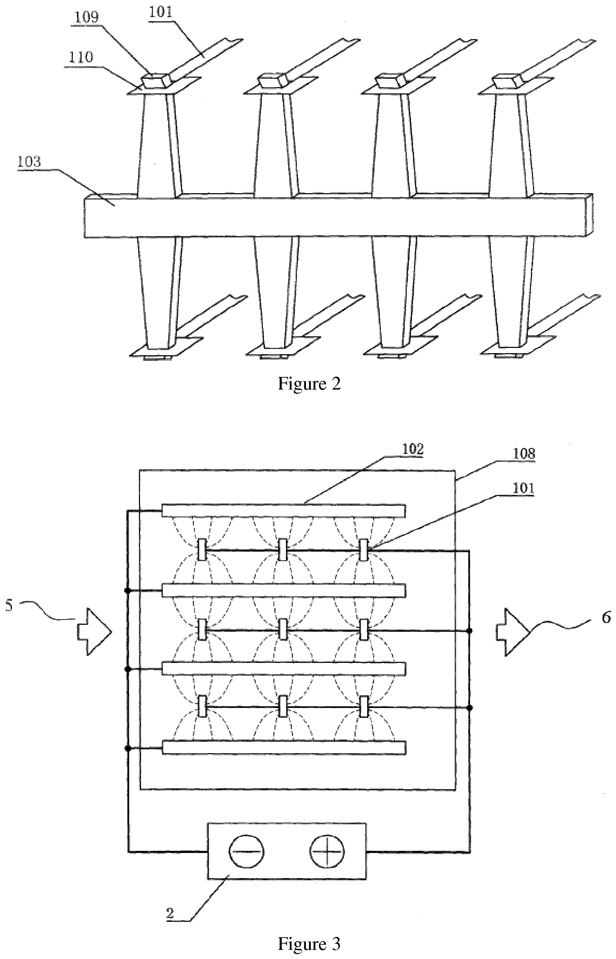

Figure 1 is a three-dimensional view with a local sectional side of one embodiment of the present invention; figure 2 is a structural view of an amplified micro-discharge preventive conductor rail in the figure 1 of the present invention.

The present invention provides a metal belt-plate structured reactor, comprising positive electrodes 101, negative electrodes 102 and a metal housing 108, wherein the positive electrode 101 is positioned at the middle of the two negative electrodes 102, the positive electrodes 101 and negative electrodes 102 are arranged along the airflow direction, and the two sides of the negative electrodes 102 are fixed on the housing 108. There are totally n groups (n is a positive integer less than 50) of positive electrodes 101, each of which is a component formed by several anti-oxidizing metal belts arrayed in parallel at an equal interval on the same plane; there are totally n+1 negative electrodes 102, each of which is made of aluminum or stainless steel plates. Several micro-discharge preventive conductor rails 103 made of aluminum bars or stainless steel bars are installed in the plasma reactor, the two ends of the positive electrode 101 are respectively fixed on the corresponding positions on the micro-discharge preventive conductor rails respectively, and the micro-discharge preventive conductor rails 103 are electrically connected by anti-oxidation leads. The negative electrode 102 may also be replaced by iron, copper or other metal plates coated with nickel and chromium on the surface, but those substitutions are inferior to the electrodes made of aluminum plates or stainless steel plates in cost performance. The corona discharge of the positive electrode 101 made of several nickel-chromium alloy belts is uniformly distributed around the positive electrode 101 along the longitudinal direction. In this embodiment, even if the reactors are different in volumes and have different positive electrode structures, the discharge distance between the positive and negative electrodes are the same in spite of such conditions as power supply configuration; the plasma which is generated by the reactor with the positive electrode 101 made of nickel-chromium alloy belts is over twice as dense as the plasma generated by the reactor with the saw-tooth or needle tip-structured positive electrode, and the ozone amount measured is 1/4 of that generated by the reactor with the saw-tooth or needle tip-structured positive electrode, meeting the provisions of the national Indoor Air Quality Standard that ozone amounts shall be less than or equal to 0.16mg/m³.

According to the present invention, a large-sized reactor for clean rooms has 30-50 groups of positive electrodes 101, a medium-sized reactor for offices has 20-40 groups of positive electrodes 101, and a small-sized reactor for households has 2-20 groups of positive electrodes 101. If applied to the super-large places such as subway stations and tunnels, several large-sized reactors can be combined together.

The housing 108 may be iron plates coated with zinc, nickel, etc. with an oxidized layer or rolled from the aluminum or stainless steel plates. It must be specified that the two sides of the housing 108 in parallel to the negative electrode 102 also function as the negative electrode, which makes the structure exquisite, simple and solid, the properties stable, and saves materials.

### Embodiment 2:

The micro-discharge preventive conductor rails 103 are provided with protruded portions 109 arrayed at an equal interval, the two ends of the positive electrode 101 are respectively fixed on the tops of the protruded portions 109 of the corresponding conduct rails; the vertically symmetrical protruded portions 109 of the micro-discharge preventive conductor rails 103 are incorporated into one group, each micro-discharge preventive conductor rail 103 has n groups of protruded portions 109, and the top of each protruded portion 109 is provided with an outward elbow. The two ends of the positive electrode 101 are respectively provided with a stainless steel connecting frame 110, of which the middle is punched into a square hole, and the elbow on the top of the protruded portion 109 is sleeved into the stainless steel connecting frame 110; the upper and lower ends, close to the edge of the reactor housing 108, of the negative electrode 102 are respectively provided with a projected negative electrode fixing pin 111 which is jointed and fastened with the corresponding groove on the reactor housing 108. The two ends of the micro-discharge conductor rail 103 are respectively provided with an insulation connection 105 fixed in the corresponding mounting hole on the reactor housing 108, and airflow channels are respectively formed on the upper and lower ends of the housing 108 along the airflow direction.

The protruded portions 109 are stainless steel elastic plates with a thickness of 0.3-1mm. During assembly, the stainless steel connecting frames 110 and the two ends of the positive electrode 101 are processed according to the designed length, and the stainless steel connecting frames 110 at the two ends are sleeved into the elbows at the protruded portions 109 of the corresponding micro-discharge preventive conductor rails.

### Embodiment 3:

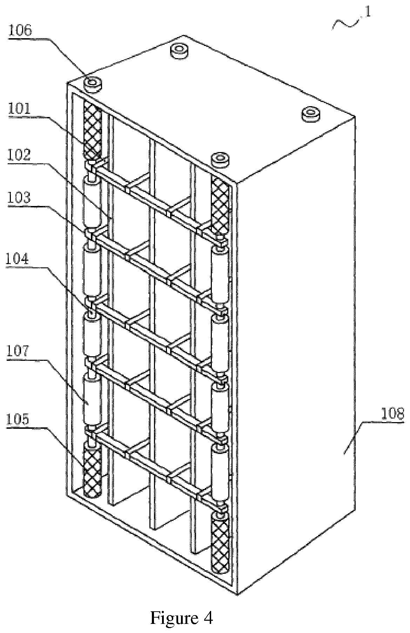

Figure 4 is a three-dimensional view of another embodiment of the present invention. In the figure, the micro-discharge preventive conductor rail 103 is provided with n groups of recessed portions which are arrayed at an equal interval, the two ends of the positive electrode 101 made of metal belts are respectively fixed into the recessed portions of the corresponding micro-discharge preventive conductor rail 103; the two ends of the micro-discharge preventive conductor rail 103 are orthogonally fixed to and electrically connected with the four positive electrode metal supports 104 on the four sides of the reactor; the outer edge of the positive electrode metal support 104 is provided with several fixing rings 107 for separating adjacent micro-discharge preventive conductor rail 103 at an interval; the upper and lower ends of each positive electrode metal support 104 are respectively provided with an insulation connection 105 fastened in the corresponding mounting hole on the reactor housing 108, and an insulation connection fixing bolt (106) fastens the insulation connection 105 on the metal reactor housing 108; the upper and lower edges, close to the reactor housing 108, of the negative electrode 102, are jointed and fastened with the corresponding positions of the reactor housing 108; and airflow channels are formed at the front and rear ends of the housing 108 in the airflow direction, respectively.

The negative electrode is made of the aluminum plate is 1-2.0mm thick and has an oxidized surface, a long service life and a good-looking appearance, or the negative electrode 102 is made of the stainless steel and is 0.5-1.5mm thick. If welding technology is used in implementation, edge titling will occur, if the bent edge of the negative electrode 102 is fastened with a screw, assembly error will be generated, and both mentioned processes are inferior to this embodiment.

### Embodiment 4:

The negative electrode 102 in the present invention is made from aluminum plates with oxidized surfaces, and the two sides of the negative electrode 102 are coated with nano-scaled TiO₂. It should be particularly highlighted that the negative electrode of the plasma reactor is made from aluminum plates with oxidized surfaces, and the upper surface is coated with TiO₂. The Al₂O₃ layer generated by the oxidation treatment is only 3-4 µm thick and has no influence on the corona discharge in the 18KV_{P-P} narrow-pulse high-voltage electric field. When the plasma reactor performs corona discharge, the blue light emitted from the reaction area contains ultraviolet light with a wavelength of 300-400 nm, and the peak value of the light intensity is 357nm. The forbidden bandwidth of TiO₂ is 3.2eV, and the wavelength threshold value of the corresponding ultraviolet ray is 387.5 nm. Experiments show that, when TiO₂ is used for sterilizing and purifying air, the optimal wavelength of the catalytic light source is less than or equal to 387.5 nm, and the peak value, namely 357nm, of the wavelength of the ultraviolet ray emitted from the reaction area meets this condition. Thus, the ultraviolet light source can be omitted, and then defects of ultraviolet lamp damage, human bodily injury and large power consumption of the ultraviolet light source are overcome.

### Embodiment 5:

The distance between the elbows on two vertically symmetrical projected portions 109 in each group is designed according to the unipolar shielding effect, and the distance between two adjacent positive electrodes 101 made of the metal belts ranges from 16-26 mm. The distance between two adjacent micro-discharge preventive conductor rails (103) with grooves is designed according to the unipolar shielding effect, the distance between two adjacent positive electrodes (101) made of the metal belts ranges from 16-26 mm, and the fixing ring (107) can be uniform in length.

The discharge distance between the positive electrode 101 and the negative electrode 102 is designed to be 8-18mm according to the electric field strength of the external high-voltage power supply.

### Embodiment 6

The positive electrode 101 in the present invention is made of anti-oxidation high-resistance electrothermal nickel-chromium alloy belts or iron-chromium-aluminum alloy materials. The metal belts forming the positive electrode 101 are 1-2mm wide and 0.05-0.2mm thick, and the thin side faces of the metal belts are aligned with and face the plane of the negative electrode 102. If the thickness is thinner, the discharge inception voltage will be lower, the concentration of the generated plasma will be higher, and the mechanical strength will be poor. A metal belt with a thickness of 0.08-0.12mm is optimal in the design. When the positive electrode made of metal belts is consumed due to discharge, the area of the discharge side face is kept unchanged, and the effective service life reaches 8-12 years. This embodiment has another technical scheme, in which the positive electrode 101 made of the iron-chromium-aluminum metal belts has a shape and dimensions identical with those of the above schemes but has a low cost, magnetism and inferior properties.

Figure 3 is a schematic view of the corona discharge of the positive electrode made of the metal belts. The reactor is externally connected with a direct current high-voltage power supply 2 and a fan, the positive electrode of the output end of the direct current high-voltage power supply is electrically connected with the positive electrode 101 of the plasma reactor 1, and the negative electrode of the output end of the direct-current high-voltage power supply is electrically connected with the negative electrode 102 of the plasma reactor 1. The left end of the reactor is the air inlet 5, the right end is the air outlet 6, and the positive electrode 101 and negative electrode 102 are arranged in parallel in the airflow direction. Viewed from the longitudinal direction, the corona discharge, on the negative electrode 102, of the positive electrode 101 made of the metal belts is more uniform than that of the saw-tooth or needle tip-structured positive electrode. The present invention can sterilize and purify the indoor air dynamically in 24 hours and has no influences on the human body or appliances and instruments.

The sterilizing factors of the present invention are low-temperature plasma and radicals generated by exciting TiO₂.

The plasma, the sterilizing factor, is a gas cloud which consists of a great amount of positive and negative charged particles and neutral particles with combined functions of the whole electric fields and charged with quasi-neutrality. The plasma can severely break down and damage the cell membranes of bacteria and break the molecular bonds of gases to generate radicals such as monatomic molecules, negative oxide ions, OH ions, free oxygen atoms and H₂O₂, which have strong activation and oxidation capabilities. It has a good effect in killing bacteria and viruses. It can also decompose macromolecular toxic organics such as the formaldehyde, benzene, radon, ammonia, carbon monoxide, smoke and TVOC and convert them into the nontoxic and scent-free inorganic substances, such as carbon and water. The internal plasma reactor has an electrostatic field which can absorb particles with a minimum particle size of 0.1µm to further purify the air. Negative oxygen ions are called "vitamins" for the air, mostly appearing near natural waterfalls and in forests. The advancement of the plasma air sterilizing and purifying technology has been approved by experts and scholars in this field, and the scientific nature and advancement of its sterilizing and purifying mechanism are incomparable; thus, this technology is internationally accepted as "one of the four technologies of environmental science in the 21 century".

By the existence of the photo-catalyst-TiO₂ and by the action of the ultraviolet rays emitted from the plasma reactor, the energy of the light source excites the gas molecules around the TiO₂ to generate radicals with strong activity. Those radicals with extremely strong activity can decompose most of the organic substances and part of the inorganic substances, thereby doubling the sterilization, deodorization and air purification effects.

The above embodiments are further descriptions of the metal belt-plate structured reactor of the present invention by means of the attached drawings and shall not be regarded as limitations of the present invention.

Within the technical concept of the present invention, those skilled in this field can make simple variations or equivalent substitutions for content of the technologies, including the materials, for preventing micro-discharge and coating TiO₂ on the negative electrode of a plasma reactor made of metal belts, all belonging to the scope of the technical scheme. The micro-discharge preventive conductor rails can be replaced by simple variations of metal tubes, but the manufacturing process is very complicated and has high cost; copper or iron plates coated with nickel or chromium may be substitutes of the negative electrodes, however they will also be inevitably oxidized lasting the end.

## Claims

1. A metal belt-plate structure reactor, comprising positive electrodes (101) having two ends and negative electrodes (102) having two sides, wherein every positive electrode (101) is located in the middle between two negative electrodes (102);**characterized in that** there are a total of n groups of positive electrodes (101), wherein n is a positive integer below 50, each of which is a component formed by several oxidation-resistant nickel-chromium alloy or iron-chromium aluminum alloy belts having a width of 1 - 2mm and a thickness of 0.05-0.2mm, with thin side faces and arrayed at an equal interval in a plane and for performing corona discharge; there are a total of n+1 negative electrodes (102) made from aluminum plates or stainless steel plates; the metal belt-plate structure reactor is also provided with several micro-discharge preventive conductor rails(103) made of aluminum bars or stainless bars; the two ends of the positive electrode (101) are respectively fixed on the corresponding positions of the conductor rails (103); the several conductor rails (103) are electrically connected by anti-oxidation leads; the thin side faces of the metal belts face and are aligned with the plane of the negative electrode (102), wherein the thin side faces of the metal belts facing the plane of the negative electrode (102) are discharge side faces which perform corona discharge; the metal belt-plate structure reactor further comprises a metal housing (108) providing an air inlet and an air outlet; the negative electrodes (102) are located in the airflow direction between the air inlet and the air outlet; the two sides of each negative electrode (102) are fixed on the housing (108).

2. The metal belt-plate structured reactor according to claim 1, wherein the conductor rail (103) is provided with n groups of protrusions (109) at an equal interval, each group consisting of an vertically upper protrusion and a vertically lower protrusion which are symmetric vertically; the two ends of the positive electrodes (101) are respectively fixed on the tops of the protrusions of the corresponding conductor rail; the top of the protrusion (109) is provided with an elbow outward from the top of the protrusion (109) ; the two ends of the positive electrode (101) are provided with a stainless steel connecting frame (110) of which the middle is punched into a square hole, and the elbow at the top of the protrusion (109) is sleeved into the square hole of the stainless steel connecting frame (110); the vertically upper and lower ends of the negative electrode (102) close to the reactor housing (108) are respectively provided with a projected negative electrode fixing bolt (111), and a position corresponding to the housing is formed with a groove for jointing and fastening; the two ends of the conductor rail (103) are respectively provided with an insulation connector (105) fixed in the corresponding mounting hole of the housing (108); and the vertically upper and lower ends of the housing (108) are provided with airflow channels along with the airflow direction between the air inlet and the air outlet.

3. The metal belt-plate structured reactor according to claim 1, wherein the conductor rail (103) is provided with n recesses at an equal interval, the two ends of the positive electrode (101) are respectively fixed on the corresponding recesses of the conductor rail (103); the two ends of the conductor rail (103) are fixed with and in electric connection with four positive electrode metal supports (104) which are orthogonally located on four sides of the reactor; the outer edge of the positive electrode metal support (104) is provided with several cylindrical fixing rings (107) for the conductor rail (103), the fixing ring (107) partitions adjacent conductor rails (103) at an equal interval; the vertically upper and lower ends of each positive electrode metal support (104) are respectively provided with an insulation connector (105) fixed with the corresponding mounting hole of the reactor housing (108) and an insulation connector fixing bolt (106) for fastening the insulation connector (105) on the metal housing (108); the vertically upper and lower sides, close to the housing (108), of the negative electrode (102) are jointed and fastened with corresponding positions of the housing (108); and the housing (108) are provided with airflow channels along with the airflow direction between the air inlet and the air outlet.

4. The metal belt-plate structured reactor according to claim 1, wherein the negative electrode (102) is made of an aluminum plate with an oxidized surface, and the two sides of the negative electrode (102) are coated with nano-scale TiO₂.

5. The metal belt-plate structured reactor according to claim 1 or 2, wherein the distance between the vertically upper and lower symmetric protrusions (109) in each group is designed in such a way that the distance between the metal belts of two adjacent positive electrodes (101) ranges from 16-26mm.

6. The metal belt-plate structured reactor according to claim 3, wherein the distance between two adjacent conductor rails (103) with recesses is designed in such a way that the distance between the metal belts of two adjacent positive electrodes (101) ranges from 16-26mm.

7. The metal belt-plate structured reactor according to claim 1, 2 or 3, wherein the discharge distance between the positive electrode (101) and negative electrode (102) is designed to be 8-18mm.

## Patentansprüche

1. Reaktor mit Metallriemen-Platten-Struktur, der positive Elektroden (101) mit zwei Enden und negative Elektroden (102) mit zwei Seiten aufweist, wobei sich jede positive Elektrode (101) in der Mitte zwischen zwei negativen Elektroden (102) befindet; **dadurch gekennzeichnet, dass** es insgesamt n Gruppen von positiven Elektroden (101) gibt, wobei n eine positive ganze Zahl unter 50 ist, von denen jede ein Bestandteil ist, der aus mehreren Riemen aus oxidationsbeständiger Nickel-Chrom-Legierung oder Eisen-Chrom-AluminiumLegierung mit einer Breite von 1-2 mm und einer Dicke von 0,05-0,2 mm ausgebildet ist, mit dünnen Seitenflächen und angeordnet in einem gleichen Abstand in einer Ebene und zum Durchführen von Koronaentladung; es insgesamt n+1 negative Elektroden (102) gibt, die aus Aluminiumplatten oder Edelstahlplatten hergestellt sind; der Reaktor mit Metallriemen-Platten-Struktur ebenso mit mehreren Mikroentladung vorbeugenden Stromschienen (103) versehen ist, die aus Aluminiumstäben oder nicht rostenden Stäben hergestellt sind; die zwei Enden der positiven Elektrode (101) jeweils an den entsprechenden Positionen der Stromschienen (103) befestigt sind; die mehreren Stromschienen (103) durch Antioxidationsanschlüsse elektrisch angeschlossen sind; die dünnen Seitenflächen der Metallriemen der Ebene der negativen Elektrode (102) zugewandt und an dieser ausgerichtet sind, wobei die dünnen Seitenflächen der Metallriemen, die der Ebene der negativen Elektrode (102) zugewandt sind, Entladungsseitenflächen sind, die Koronaentladung durchführen; der Reaktor mit Metallriemen-Platten-Struktur ferner ein Metallgehäuse (108) aufweist, das einen Lufteinlass und einen Luftauslass bereitstellt; die negativen Elektroden (102) sich in der Luftströmungsrichtung zwischen dem Lufteinlass und dem Luftauslass befinden; die zwei Seiten jeder negativen Elektrode (102) am Gehäuse (108) befestigt sind.

2. Reaktor mit Metallriemen-Platten-Struktur nach Anspruch 1, wobei die Stromschiene (103) mit n Gruppen von Vorsprüngen (109) in einem gleichen Abstand versehen ist, wobei jede Gruppe aus einem vertikal oberen Vorsprung und einem vertikal unteren Vorsprung besteht, die vertikal symmetrisch sind; die zwei Enden der positiven Elektroden (101) jeweils an den Oberseiten der Vorsprünge der entsprechenden Stromschiene befestigt sind; die Oberseite des Vorsprungs (109) mit einem Bogenstück nach außen von der Oberseite des Vorsprungs (109) versehen ist; die zwei Enden der positiven Elektrode (101) mit einem Edelstahlverbindungsrahmen (110) versehen sind, von dem die Mitte in ein Vierkantloch gestanzt ist, und das Bogenstück an der Oberseite des Vorsprungs (109) in das Vierkantloch des Edelstahlverbindungsrahmens (110) gestülpt ist; die vertikal oberen und unteren Enden der negativen Elektrode (102) nahe dem Reaktorgehäuse (108) jeweils mit einem hervorstehenden Befestigungsbolzen (111) der negativen Elektrode versehen sind und eine dem Gehäuse entsprechende Position mit einer Nut zum Verbinden und Befestigen ausgebildet ist; die zwei Enden der Stromschiene (103) jeweils mit einem Isolierverbinder (105) versehen sind, der im entsprechenden Montageloch des Gehäuses (108) befestigt ist; und die vertikalen oberen und unteren Enden des Gehäuses (108) zusammen mit der Luftströmungsrichtung zwischen dem Lufteinlass und dem Luftauslass mit Luftströmungskanälen versehen sind.

3. Reaktor mit Metallriemen-Platten-Struktur nach Anspruch 1, wobei die Stromschiene (103) mit n Aussparungen in einem gleichen Abstand versehen ist, wobei die zwei Enden der positiven Elektrode (101) jeweils an den entsprechenden Aussparungen der Stromschiene (103) befestigt sind; die zwei Enden der Stromschiene (103) mit vier Metallträgern (104) der positiven Elektrode befestigt sind und mit diesen in elektrischer Verbindung stehen, die sich rechtwinklig an vier Seiten des Reaktors befinden; die Außenkante des Metallträgers (104) der positiven Elektrode mit mehreren zylinderförmigen Befestigungsringen (107) für die Stromschiene (103) versehen sind, wobei der Befestigungsring (107) benachbarte Stromschienen (103) in einem gleichen Abstand abteilt; die vertikalen oberen und unteren Enden jedes Metallträgers (104) der positiven Elektrode jeweils mit einem Isolierverbinder (105), der mit dem entsprechenden Montageloch des Reaktorgehäuses (108) befestigt ist, und einem Befestigungsbolzen (106) des Isolierverbinders zum Befestigen des Isolierverbinders (105) auf dem Metallgehäuse (108) versehen sind; die vertikal oberen und unteren Seiten, nahe dem Gehäuse (108), der negativen Elektrode (102) mit entsprechenden Positionen des Gehäuses (108) verbunden und befestigt sind; und das Gehäuse (108) zusammen mit der Luftströmungsrichtung zwischen dem Lufteinlass und dem Luftauslass mit Luftströmungskanälen versehen sind.

4. Reaktor mit Metallriemen-Platten-Struktur nach Anspruch 1, wobei die negative Elektrode (102) aus einer Aluminiumplatte mit einer oxidierten Oberfläche hergestellt ist und die zwei Seiten der negativen Elektrode (102) mit TiO₂ im Nanobereich beschichtet sind.

5. Reaktor mit Metallriemen-Platten-Struktur nach Anspruch 1 oder 2, wobei die Entfernung zwischen den vertikal oberen und unteren symmetrischen Vorsprüngen (109) in jeder Gruppe so ausgelegt ist, dass die Entfernung zwischen den Metallriemen von zwei benachbarten positiven Elektroden (101) von 16 bis 26 mm reicht.

6. Reaktor mit Metallriemen-Platten-Struktur nach Anspruch 3, wobei die Entfernung zwischen zwei benachbarten Stromschienen (103) mit Aussparungen so ausgelegt ist, dass die Entfernung zwischen den Metallriemen von zwei benachbarten positiven Elektroden (101) von 16 bis 26 mm reicht.

7. Reaktor mit Metallriemen-Platten-Struktur nach Anspruch 1, 2 oder 3, wobei die Entladungsentfernung zwischen der positiven Elektrode (101) und der negativen Elektrode (102) ausgelegt ist, dass sie 8-18 mm beträgt.

## Revendications

1. Réacteur à structure de courroies-plaques métalliques, le réacteur comprenant des électrodes positives (101) comportant deux extrémités et des électrodes négatives (102) comportant deux côtés, chaque électrode positive (101) étant située au milieu entre deux électrodes négatives (102) ; **caractérisé en ce que** : il y a un total de n groupes d'électrodes positives (101), n étant un nombre entier positif inférieur à 50, chaque électrode positive étant un élément formé à partir de plusieurs courroies en alliage nickel-chrome ou en alliage fer-chrome aluminium résistant à l'oxydation, présentant une largeur comprise entre 1 et 2 mm et une épaisseur comprise entre 0,05 et 0,2 mm, dotées de faces latérales minces et agencées à intervalles égaux dans un plan, et permettant d'effectuer une décharge par effet corona ; il y a un total de n+1 électrodes négatives (102) formées à partir de plaques d'aluminium ou de plaques d'acier inoxydable ; le réacteur à structure de courroies-plaques métalliques est muni également de plusieurs rails conducteurs préventifs de micro-décharge (103), formés à partir de barres d'aluminium ou de barres inoxydables ; les deux extrémités de l'électrode positive (101) sont fixées respectivement sur les positions correspondantes des rails conducteurs (103) ; les plusieurs rails conducteurs (103) sont connectés électriquement par des fils anti-oxydation ; les faces latérales minces des courroies métalliques sont orientées vers le plan de l'électrode négative (102) et alignées avec ledit plan, les faces latérales minces des courroies métalliques orientées vers le plan de l'électrode négative (102) étant des faces latérales de décharge effectuant une décharge par effet corona ; le réacteur à structure de courroies-plaques métalliques comprend en outre une enveloppe métallique (108) fournissant une entrée d'air et une sortie d'air; les électrodes négatives (102) sont situées dans la direction d'écoulement d'air entre l'entrée d'air et la sortie d'air ; les deux côtés de chaque électrode négative (102) sont fixés sur l'enveloppe (108).

2. Le réacteur à structure de courroies-plaques métalliques selon la revendication 1, dans lequel le rail conducteur (103) est muni de n groupes de saillies (109) à intervalles égaux, chaque groupe consistant en une saillie verticalement supérieure et une saillie verticalement inférieure qui sont symétriques verticalement ; les deux extrémités des électrodes positives (101) sont fixées respectivement sur les parties supérieures des saillies du rail conducteur correspondant ; la partie supérieure de la saillie (109) est munie d'un coude vers l'extérieur à partir de la partie supérieure de la saillie (109) ; les deux extrémités de l'électrode positive (101) sont munies d'un cadre de liaison en acier inoxydable (110) dont le centre est perforé pour former un trou carré, et le coude au niveau de la partie supérieure de la saillie (109) est emmanché dans le trou carré du cadre de liaison en acier inoxydable (110) ; les extrémités verticalement supérieure et inférieure de l'électrode négative (102) à proximité de l'enveloppe (108) du réacteur sont munies respectivement d'un boulon (111) de fixation d'électrode négative en saillie, et une position correspondant à l'enveloppe est pourvue d'une rainure permettant l'assemblage et la fixation ; les deux extrémités du rail conducteur (103) sont munies respectivement d'un raccord isolant (105) fixé dans le trou de montage correspondant de l'enveloppe (108) ; et les extrémités verticalement supérieure et inférieure de l'enveloppe (108) sont munies de canaux d'écoulement d'air le long de la direction d'écoulement d'air entre l'entrée d'air et la sortie d'air.

3. Le réacteur à structure de courroies-plaques métalliques selon la revendication 1, dans lequel le rail conducteur (103) est muni de n évidements à intervalles égaux, les deux extrémités de l'électrode positive (101) étant fixées respectivement sur les évidements correspondants du rail conducteur (103) ; les deux extrémités du rail conducteur (103) sont fixées à, et en connexion électrique avec, quatre supports métalliques (104) d'électrode positive situés orthogonalement sur quatre côtés du réacteur ; le bord externe du support métallique (104) d'électrode positive est muni de plusieurs anneaux de fixation cylindriques (107) pour le rail conducteur (103), l'anneau de fixation (107) séparant des rails conducteurs (103) adjacents à intervalles égaux; les extrémités verticalement supérieure et inférieure de chaque support métallique (104) d'électrode positive sont munies respectivement d'un raccord isolant (105) fixé au trou de montage correspondant de l'enveloppe (108) du réacteur et d'un boulon (106) de fixation de raccord isolant permettant de fixer le raccord isolant (105) sur l'enveloppe métallique (108); les côtés verticalement supérieur et inférieur, à proximité de l'enveloppe (108), de l'électrode négative (102) sont assemblés et fixés à des positions correspondantes de l'enveloppe (108) ; et l'enveloppe (108) est munie de canaux d'écoulement d'air le long de la direction d'écoulement d'air entre l'entrée d'air et la sortie d'air.

4. Le réacteur à structure de courroies-plaques métalliques selon la revendication 1, dans lequel l'électrode négative (102) est formée à partir d'une plaque d'aluminium dotée d'une surface oxydée, et les deux côtés de l'électrode négative (102) sont revêtus de TiO₂ nanométrique.

5. Le réacteur à structure de courroies-plaques métalliques selon les revendications 1 ou 2, dans lequel la distance entre les saillies symétriques verticalement supérieure et inférieure (109) dans chaque groupe est conçue de telle façon que la distance entre les courroies métalliques de deux électrodes positives (101) adjacentes varie entre 16 et 26 mm.

6. Le réacteur à structure de courroies-plaques métalliques selon la revendication 3, dans lequel la distance entre deux rails conducteurs (103) adjacents dotés d'évidements est conçue de telle façon que la distance entre les courroies métalliques de deux électrodes positives (101) adjacentes varie entre 16 et 26 mm.

7. Le réacteur à structure de courroies-plaques métalliques selon les revendications 1, 2 ou 3, dans lequel la distance de décharge entre l'électrode positive (101) et l'électrode négative (102) est conçue pour être comprise entre 8 et 18 mm.
